# EUROPEAN PATENT SPECIFICATION

(11) **EP 3 740 769 B1**
(45) Date of publication and mention of the grant of the patent: **26.03.2025**
(21) Application number: 19706051.0
(22) Date of filing: 10.01.2019
(51) Int. Cl.: G01R 19/165, H02J 7/00, G01R 31/371, G01R 31/3835, A61L 9/03, G01L 23/08, B60H 3/00

(54) **ELECTRONIC DEVICES FOR USE IN A VEHICLE AND METHODS OF OPERATING THE SAME**
ELEKTRONISCHE VORRICHTUNGEN ZUR VERWENDUNG IN EINEM FAHRZEUG UND VERFAHREN ZUM BETRIEB DAVON
DISPOSITIFS ÉLECTRONIQUES DESTINÉS À ÊTRE UTILISÉS DANS UN VÉHICULE ET LEURS PROCÉDÉS DE FONCTIONNEMENT

(30) Priority: 17.01.2018 US 201815873681
(43) Date of publication of application: 25.11.2020
(73) Proprietor: S.C. Johnson & Son, Inc., Racine, WI 53403 (US)
(72) Inventor: WITCRAFT, Craig J., Vernon Hills, Illinois 60061 (US); FATERIOUN, Kamran, New Berlin, Wisconsin 53146 (US)
(74) Representative: Murgitroyd & Company
(86) International application number: PCT/US2019/013046
(87) International publication number: WO 2019/143518

(56) References cited:
- EP-A1- 2 985 738
- DE-C1- 3 841 769
- US-A1- 2004 229 110
- US-A1- 2016 161 367
- US-A1- 2017 252 476
- US-B2- 8 437 908

## Description

### BACKGROUND

### 1. Field of the Invention

The present disclosure generally relates to automotive applications, and more specifically, to electronic devices for use in a vehicle and methods of operating the same.

### 2. Description of the Background of the Invention

Many modern electronic devices, including cell phones, tablets, laptops, and others, are often recharged using vehicle power. In addition, individuals often utilize personal volatile emitting devices in their vehicles, such as air fresheners, odor eliminators, or other devices designed to assist in eliminating pests, cleaning, or freshening the surrounding environment. In particular, many volatile emitting devices include a heat source or fan that that assist in the dispersion or release of the volatile from a cartridge. To operate heating elements, fans, and other circuitry, volatile emitting devices and other devices are designed to utilize power from the 12V accessory outlet of the vehicle.

However, many electronic devices may continue to draw power even after the vehicle has been turned off. If left connected for an extended period of time, this can result in unwanted battery drain and perhaps vehicle incapacitation, particularly for devices that draw high power. In the case of personal volatile emitting devices, volatiles may also be depleted prematurely. To avoid such difficulties, some technologies have attempted to include capabilities for determining whether a vehicle is on or off by incorporating vibration sensors, for instance. However, vibration sensors can produce errors due to their inability to discriminate vibrations unrelated to vehicle operation. In addition, vibration sensors increase device cost and design complexity.

Other technologies have been used to determine an operational state of a vehicle. For example, US 2016/161367 A1 discloses a device and method for detecting a vehicle engine state that includes a power input terminal and a power monitoring unit. The power input terminal is electrically connected to a battery. The power monitoring unit is electrically connected to the power input terminal and configured to detect a battery voltage of the battery and to update a standby threshold voltage predefined in the power monitoring unit according to a standby level of the battery in a steady state whenever an engine shuts down. The power monitoring unit is configured to compare the detected battery voltage and the updated standby threshold voltage, and to determine if the engine is activated according to a result of the comparison. US 2004/229110 A1 discloses a low voltage auto-interruption device of a car battery which comprises a voltage detection and interruption device. DE 38 41 769 C1 discloses a circuit for ensuring the provision of starting energy in motor vehicles with internal combustion engines, which does not draw any current when consumers are switched off. US 2017/252476 A1 discloses a volatile material dispenser that may include an electric power sensor to detect a change of voltage from the power source when a user turns the automobile on or off.

Therefore, a need exists for a low cost, reliable way of controlling electronic devices based on the operational state of a vehicle.

### SUMMARY

The present disclosure overcomes drawbacks of previous technologies. Features and advantages of the present disclosure will become apparent from the following description.

In one aspect of the present disclosure, a method for controlling an electronic device for use in a vehicle according to claim 1 is provided.

In another aspect of the present disclosure, an electronic device for use in a vehicle according to claim 6 is provided.

In yet another aspect of the present disclosure, a volatile emitting device for use in a vehicle according to claim 12 is provided.

### BRIEF DESCRIPTION OF THE DRAWINGS

FIG. 1 is a flowchart setting forth steps of a process, in accordance with aspects of the present disclosure;
FIG. 2 is another flowchart setting forth steps of a process, in accordance with aspects of the present disclosure;
FIG. 3 is a schematic diagram of an electronic device, in accordance with aspects of the present disclosure;
FIG. 4 is a schematic diagram illustrating one embodiment of the electronic device shown in FIG. 3;
FIG. 5 is a schematic diagram of another embodiment, in accordance with aspects of the present disclosure;
FIG. 6 is perspective view of an example volatile emitting device, in accordance with aspects of the present disclosure; and
FIG. 7 is a schematic diagram of an example electrical assembly implemented in the device shown in FIG. 6.

Other aspects and advantages of the present invention will become apparent upon consideration of the following detailed description, wherein similar structures have similar reference numerals.

### DETAILED DESCRIPTION

The present disclosure is directed to a novel approach for controlling electronic devices for use in a vehicle. In particular, methods for operating devices based on the operational state of the vehicle are provided. As will become apparent from the description below, methods described herein may be advantageously implemented for a wide variety of electronic devices commonly used in vehicles, including volatile emitting devices, cell phones, tablets, laptops, GPS devices, navigation units, cameras, FM broadcasters, Bluetooth devices, AC adapters, video games, air compressors, and many others.

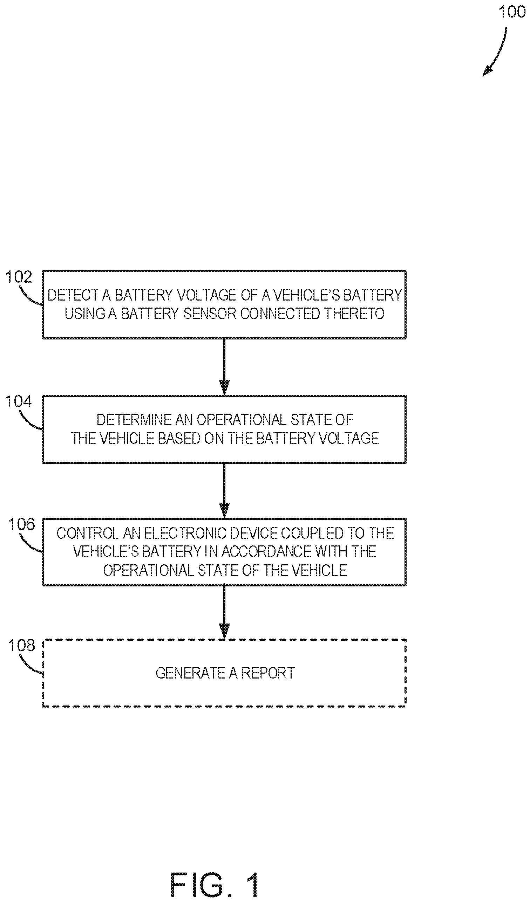

Referring to FIG. 1, steps of a process 100 are shown, which may be carried out using any suitable device, apparatus, or system, including devices described in the present disclosure. Steps of the process 100 may be implemented as a program, firmware, or executable instructions stored in non-transitory computer readable media.

The process 100 may begin at process block 102 by detecting a battery voltage of a vehicle's battery. The battery voltage is detected using a battery sensor connected or connectable to the vehicle's battery. The battery sensor may be electrically connected, but need not be physically connected, to the vehicle's battery. As will be described, in some embodiments, the battery sensor may be incorporated into, or may be part of, various electronic devices, such as portable electronic devices, volatile emitting devices, and others. In other embodiments, the battery sensor may be part of the vehicle circuitry.

In some aspects, the battery voltage detected by the battery sensor may be sampled over a predefined period of time, either intermittently or periodically using a predefined sampling frequency. By way of example, the predefined period of time may range approximately between a few seconds and 30 minutes. However, in some implementations, the period of time may be less than a second or longer than 30 minutes. Also, the predefined sampling frequency may range between approximately 0.1 Hz, or less and 10,000 Hz, or more. The battery voltage may then be analyzed by a processor to generate various information therefrom. Such information may include, for instance, maximum battery voltage, minimum battery voltage, average battery voltage, battery voltage standard deviation, rate of change of the battery voltage, and so forth.

Then, at process block 104, an operational state of the vehicle is determined based on the battery voltage detected. The battery voltage is compared to one or more predefined thresholds. In addition, information obtained from the analyzed battery voltage samples may be compared to predefined signatures. Such thresholds and signatures may be obtained by measuring battery voltage in various vehicle operation scenarios.

In general, the battery voltage level depends on whether the vehicle's engine is on or off. As such, battery voltage may be used as an indicator of the operational state of the vehicle's engine. Specifically, when the engine is off, battery voltage can range up to (or approximately to) a nominal voltage of 12.6V, depending upon the battery's age and charge level. When the engine is on, the vehicle alternator charges the battery, and the battery voltage rises to the operating voltage of the vehicle alternator, which is typically greater than 13.0V. Therefore, a battery voltage above a threshold of approximately 13.0V may indicate an "ON" state of the vehicle's engine.

However, some "smart" alternators can also drive a vehicle battery at an operating voltage below 13.0V. For instance, the battery voltage may be close to a voltage corresponding to the engine being off. Therefore, a measurement below 13.0V might not conclusively indicate whether the vehicle is in an "ON" or "OFF" state. To differentiate between the two operational states, a load may be applied to the battery for a period of time while the battery voltage is monitored. Specifically, a change in the battery voltage is then used to determine the state. For instance, if the battery voltage rises, does not change at all, does not appreciably change, or drops slower than a predetermined rate, then the battery is charging, and the vehicle is in an "ON" state. Otherwise, if the battery voltage drops faster than the predetermined rate, the battery is not charging and the vehicle's engine is in an "OFF" state.

The period of time for monitoring the battery voltage may vary from a few seconds, or less, to 30 minutes, or more. In some aspects, the period of time may depend on the load being applied, the sampling rate of the battery voltage, as well as the time required to observe any appreciable changes in the battery voltage, if they should occur. In other aspects, the period of time may also depend on the type of vehicle. For instance, a hybrid vehicle may automatically turn off its engine at stop lights, railroad crossings, and so on, to conserve resources. Therefore, a period of time sufficient to distinguish operational states in such scenarios would be advantageous. This would allow operation of an electronic device to continue without interruption.

Although description above makes reference to the state of the vehicle's engine, the operational state of the vehicle determined at process block 104 may also refer to a state of the ignition, for instance, in accordance with a position of ignition key.

Referring again to FIG. 1, an electronic device coupled to the vehicle's battery is then controlled at process block 106 in accordance with the operational state of the vehicle. The process of controlling the device may include adapting one or more functions or operational aspects of the device. In some implementations, those functions or operational aspects of the device requiring substantial power from the vehicle's battery may be turned off or converted to a low-power mode if the vehicle is in an "OFF" state or the vehicle's battery is discharged below a predetermined threshold. In one example, charging of the electronic device using the vehicle's battery may be modified or interrupted if the vehicle is determined to be in an "OFF" state. In another example, an electronic device, or various components therein, relying on power from the vehicle's battery may be turned off or entered into a low-power mode. Specifically, power to a heating element, fan or USB port of a volatile emitting device may be interrupted or reduced. It may be appreciated that these examples are not limiting, and a wide variety of a device's functions or operational aspects may be controlled based on what the operational state of the vehicle is determined to be.

In some aspects, a report may be generated, as indicated by process block 108. The report may be in any form and include any information. The report may be provided to an output, and/or stored in a memory. For example, the report may be provided using a display and/or LEDs, and so forth, and indicate various battery voltages detected, a determined operational state of the vehicle, a condition of a device, a communication link, and other data or information.

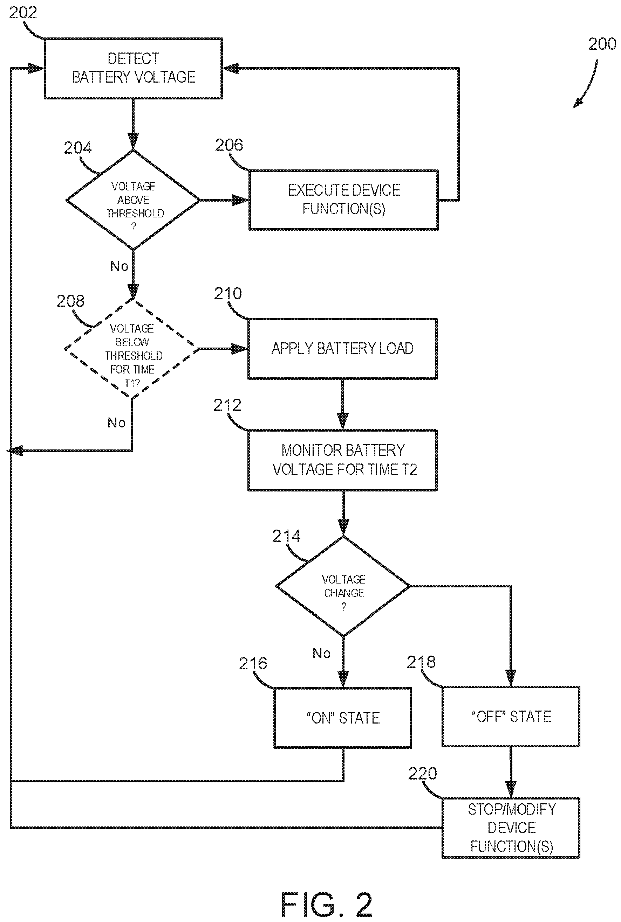

Turning now to FIG. 2, another flowchart setting forth steps of a process 200, in accordance with aspects of the present disclosure, is shown. The process 200 may be carried out using any suitable device, apparatus, or system, including devices described in the present disclosure. Steps of the process 200 may be implemented as a program, firmware, or executable instructions stored in non-transitory computer readable media.

The process 200 may begin at process block 202 by detecting the battery voltage of a vehicle. As described, battery voltage is detected using a battery sensor coupled to the vehicle's battery and may be sampled intermittently or periodically over a predetermined period of time. Then, a determination is made whether the battery voltage is above a threshold, as indicated by decision block 204. As an example, the threshold may be approximately 13.0V, although other thresholds may be possible.

The determination at decision block 204 can be made based on one or more battery voltage samples obtained at process block 202, as described. In one example, the determination may be made based on whether an average battery voltage is above the threshold. If the battery voltage, or average battery voltage, is above the threshold, one or more functions or operational aspects of an electronic device coupled to the battery may be executed, as indicated by process block 206. Example functions may include charging the device, operating a heating element, operating a fan, and so on.

If the battery voltage, or average battery voltage, is below the threshold, another determination may be optionally made, as indicated by decision block 208. Specifically, it may be determined whether the battery voltage, or average battery voltage, is below the threshold for a time T1. In one example, T1 may be approximately 30 minutes, although T1 could be longer or shorter, and may depend on the sampling rate of the battery voltage, vehicle type, and other factors, as mentioned. This could help avoid, for instance, incorrect determinations based on voltage spikes or other transients. If the battery voltage, or average battery voltage, persists below the threshold for the time T1, a load is then applied to the battery, as indicated by process block 210. Otherwise, process block 202 may be repeated, as desired.

The battery voltage may then be monitored for a time T2 at process block 212. For example, the battery voltage may be monitored for approximately one minute, or less, or more. Then, a determination is made at decision block 214 with respect to any changes in the battery voltage. Specifically, if the battery voltage, or average battery voltage, rises, does not appreciably change, does not change at all, or drops slower than a predetermined rate as a result of the applied load, then the battery is charging. Thus, the vehicle is in an "ON" state, as indicated by process block 216. Otherwise, if the battery voltage drops faster than the predetermined rate, then the battery is not charging, and the vehicle is in an "OFF" state, as indicated by process block 218.

In some preferred implementations, the applied load and/or duration T2 for monitoring the battery voltage at process blocks 210 and 212 may be configured such that a determination at decision block 214 can be made without adversely affecting the battery. For instance, the applied load and/or duration T2 may be selected to induce a detectable change in the battery voltage when the vehicle is in an "OFF" state, and without significantly discharging the battery.

As indicated by process block 220, one or more device functions or operational aspects may be stopped or modified if the vehicle is determined to be in an "OFF" state. In one non-limiting example, operation of a heating element or fan may be stopped or reduced. In another non-limiting example, the device may be placed into a low-power state or device charging may be interrupted.

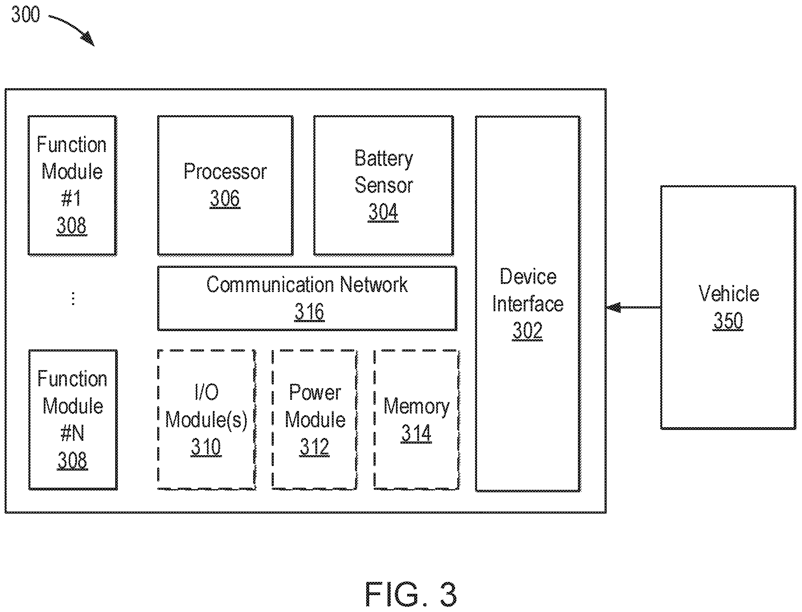

Turning now to FIG 3, a schematic diagram of an electronic device 300, in accordance with aspects of the present disclosure, is shown. As illustrated, the device 300 is configured to cooperate with a vehicle 350. In general, the vehicle 350 may include an automobile, an aircraft, a boat, a drone, a golf cart, and others.

As shown, the device 300 may generally include a device interface 302, a battery sensor 304, a processor 306, and a number of function modules as 308. The device 300 may optionally include one or more input/output (I/O) modules 310, a power module 312, a memory 314, as well as other elements or circuitry. A communication network 316 may also be included in the device 300 and configured to facilitate the exchange of data, signals, and other information between the various elements of the device 300.

The device interface 302 may be configured to exchange data, signals, and other information with a variety of devices and/or a system. As shown in FIG. 3, in some embodiments, the device interface 302 allows communication of signals, data, and other information with the vehicle 350. In particular, the device interface 302 may be configured to provide an electrical wired or wireless connection between the battery of the vehicle 350 and various components in the device 300, such as the battery sensor 304, the power module 312, and others. In one example, the device interface 302 may include one or more electrical connectors configured to make an electrical contact with the vehicle 350. In some embodiments, the electrical connectors are configured to couple to a power socket of the vehicle 350, thereby electrically coupling or connecting the device 300, and various components therein, to the vehicle's battery.

The battery sensor 304 is in communication with the device interface 302 and configured to detect the battery voltage of the vehicle 350. In some implementations, the battery sensor 304 may include a voltage detector configured to at least detect voltages in a range applicable to vehicle battery voltages. Example voltage detectors may include voltmeters, data acquisition cards, Arduino boards, and other analog and/or digital circuitry. In addition, in some implementations, the battery sensor 304 may include a variety of other electronic components and hardware for acquiring, pre-processing, and/or modifying signals (e.g. voltages or currents) received via the device interface 302. In some implementations, such electronic components and hardware may be configured to sample, amplify, filter, scale, and digitize signals received by the battery sensor 304. The battery sensor 304 may also include various protective circuitry, fault detectors, switches, and so on, configured for protecting sensitive components in the device 300.

In addition to being configured to carry out various processes of the device 300, the processor 306 is configured to execute steps, in accordance with methods of the present disclosure. Specifically, the processor 306 may include one or more processors or processing units configured to carry out steps to determine a state of operation of the vehicle 350 based on the battery voltage detected. The processor 306 also controls operation of the device 300 in accordance with the operational state, as described. In some aspects, the processor 306 may also determine and generate a report indicating a state of a vehicle's battery (e.g., discharged state, charging state, full state, and so on), as well as other information related to battery voltage detected and a vehicle's operational state(s). To do so, the processor 306 may execute hardwired instructions or programming. As such, the processor 306, or various processing units therein, may therefore be application-specific due to the hardwired instructions or programming therein. Alternatively, the processor 306 may execute non-transitory instructions stored in the memory 314, as well as instructions received via input. By way of example, the processor 306 may include one or more general-purpose programmable processors, such as central processing units ("CPUs"), graphical processing units ("GPUs"), microcontrollers, and the like.

In some aspects, the processor 306 may control one or more function modules 308 in the device 300. As shown in FIG. 3, the device 300 may include one or more function modules 308 that are configured to carry out specific functions in the device 300. In one non-limiting example, one function module 308 may be configured to control a heating element, or a fan in the device 300. In another non-limiting example, another function module 308 may be configured to control the charging of a battery in the device 300. In yet another non-limiting example, another function module 308 may be configured to control charging of an external battery connected to the device 300, where the external battery (e.g., of a cell phone or tablet) is connected via a USB port on the device 300. In yet another non-limiting example, yet another function module 308 may be configured to control the power module 312 to modify a power state of the device 300 (e.g., normal power state, low-power state, sleep state, and so forth). Alternatively, the power module 312 may be controlled directly by the processor 306. In yet another non-limiting example, another function module 308 may communicate with the I/O module(s) 310 to provide a report indicating a condition of the device 300. To this end, the one or more function modules 308 may include a variety of elements, circuitry, and hardware, including various signal sources, signal processors, integrated circuits, digital-to-analog ("DAC") converters, analog-to-digital converters ("ADC"), pulse width modulation ("PWM") generators, analog/digital voltage switches, analog/digital pots, relays, and other electrical components.

As mentioned, the device 300 may optionally include I/O module(s) 310 configured to receive a variety of data, information, as well as selections, and operational instructions from an operator. To this end, the I/O module(s) 310 may also include various drives, ports, receptacles, and elements for providing input, including a touchpad, touch screen, buttons, switches, toggles, flash-drives, USB ports/drives, CD/DVD drives, communication ports, modules, and connectors, and so on. The I/O module(s) 310 may also be configured to provide a report by way of various output elements, including screens, LEDs, LCDs, alarm sources, and so on.

The power module 312 may be configured to provide power to various elements of the device 300. In some implementations, the power module 312 may power the various elements by way of a vehicle battery. Additionally, or alternatively, the power module 312 may include an internal source of power, including a rechargeable or replaceable battery. To this end, the power module 312 may control the charging of the battery, as well as dissemination of power provided by the vehicle 350 and/or battery. In some implementations, the power module 312 may also provide power to an external device, or control the charging of the external device, connected to the device 300 using a USB port, for example.

The memory 314 may store a variety of information and data, including, for example, operational instructions, data, and so on. In some aspects, the memory 314 may include non-transitory computer readable media having instructions executable by the processor 306. The memory 314 may also store data corresponding to detected battery voltages and information generated therefrom, including battery states, vehicle operational states, and so on.

The communication network 316 may include a variety of communication capabilities and circuitry, including various wiring, components, and hardware for electronic, radiofrequency ("RF"), optical, and other communication methods. By way of example, the communication network 316 may include parallel buses, serial buses, and combinations thereof. Example serial buses may include serial peripheral interface (SPI), I2C, DC-BUS, UNI/O, 1-Wire, and others. Example parallel buses may include ISA, ATA, SCSI, PIC, IEEE, and others.

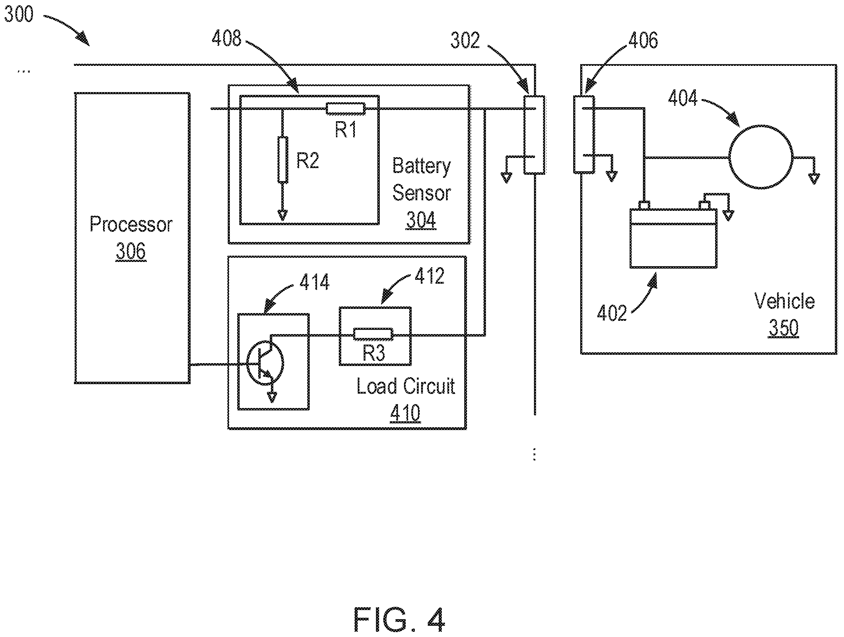

One embodiment of the device 300 described above is illustrated in FIG. 4. Specifically, the battery sensor 304 may be electrically coupled to the vehicle battery 402 and vehicle alternator 404 via the device interface 302 and a vehicle interface 406. Such coupling may be achieved using a wired, and optionally grounded, connection, as shown in FIG. 4, as well as a wireless connection. In one implementation, the vehicle interface 406 may include an accessory outlet of the vehicle 350 (e.g., a 12V power socket) and the device interface 302 may include a plug configured to electrically and mechanically engage the accessory outlet. As shown, the battery sensor 304 may include a voltage divider 408 having a first resistor R1 and a second resistor R2. Selection of R1 and R2 may depend upon the battery voltage supplied by the vehicle battery 402, as well as on the specifics of the processor 306. For example, R1 and R2 may depend upon the voltage range capability of the processor 306.

As shown, the processor 306 is also connected to a load circuit 410 configured to apply a load to the vehicle battery 402. Specifically, the load circuit 410 may include a load 412 and a switch 414 configured to engage the load 414, as directed by the processor 306. In some implementations, the load 412 may be a resistor R3 (e.g., a heating element or resistive wire) and the switch 414 may be a transistor element. It may be readily appreciated, however, that the load 412 and switch 414 may include any elements or hardware designed to achieve the same or a similar functionality. For example, the load 412 may include any element or component that can draw power from the vehicle battery 402, and the switch 414 may include any element or component that can engage the load 412 to the vehicle battery 402. In some implementations, the load circuit 410 may include, or be part of, a function module 308, as described with reference to FIG. 3. For example, the load circuit 410 may be an electric circuit having a heating element configured to control or assist in the dispensing of a volatile material.

Referring again to FIG. 4, the processor 306 then receives, samples, and processes signals (e.g. voltage signals) from the vehicle battery 402 by way of the battery sensor 304, to determine an operational state of the vehicle 350. As described, the processor 306 also controls the load circuit 410 in determining the operational state of the vehicle 350. The processor 306 then controls the device 300, and various function modules 308 therein, as described with reference to FIG. 3.

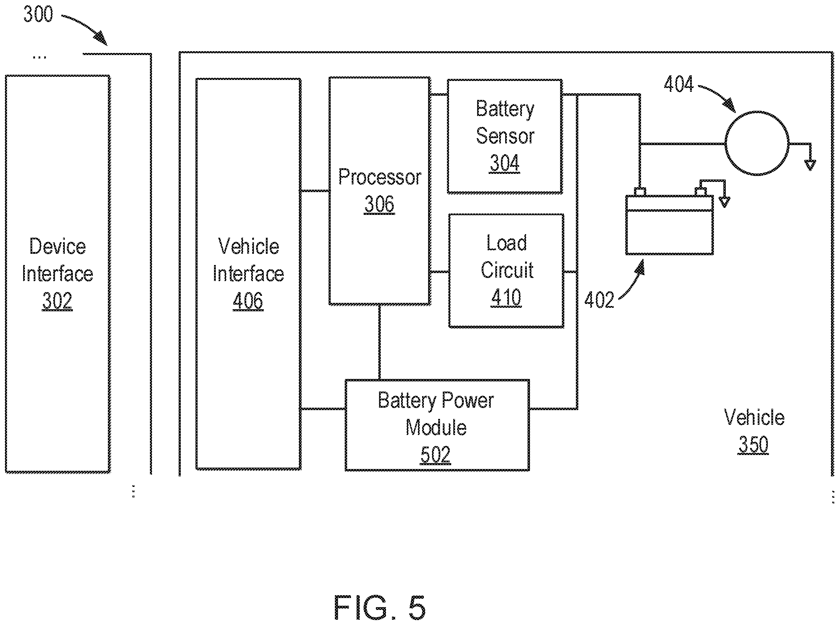

In some embodiments, as illustrated in FIG. 5, the battery sensor 304, processor 306, and load circuit 410 may be incorporated in the vehicle 350, rather than the device 300. As shown, the processor 306 may also be connected to a battery power module 502 configured to provide power to the device 300 by way of the vehicle interface 406 and device interface 302. As described, the processor 306 is configured to determine an operational state of the vehicle 350 based on battery voltages detected using the battery sensor 304. The processor 306 may then communicate with the battery power module 502 to control power provided by the vehicle battery 402 to the device 300. For example, if it is determined that the vehicle is in an "OFF" state, or the battery is discharged below a predetermined threshold, the processor 306 may generate and send control signals to the battery power module 502 to interrupt power available to the device 300 at the vehicle interface 406. The processor 306 may also generate a report and communicate the report via the vehicle interface 406. In some aspects, the report may include an indication of battery state or vehicle operational state, as well as other information, such as operational instructions for the device 300. For example, the operational instructions may include instructions for the device 300 to enter a low-power mode.

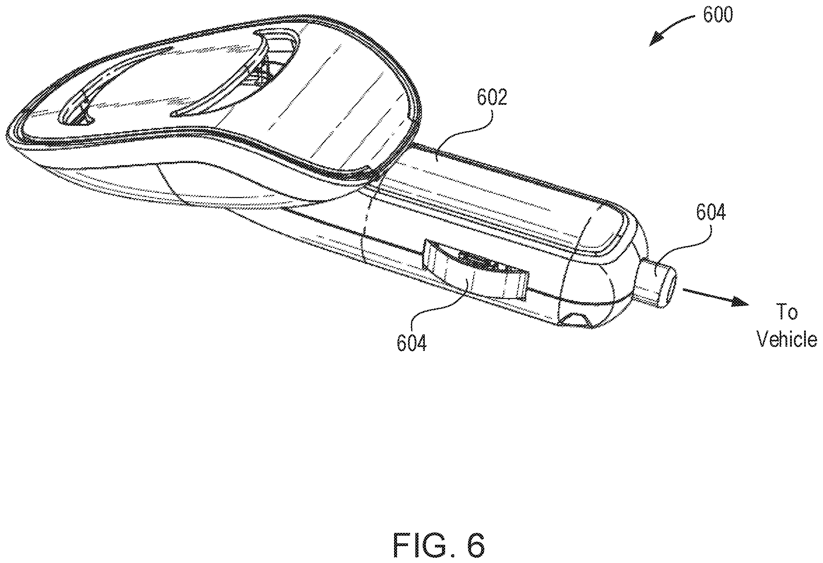

FIG. 6 shows one embodiment of a volatile material device 600 for use in a vehicle, in accordance with aspects of the present disclosure. As appreciated from the description above, FIG. 6 is provided for purposes of illustrating devices and methods, and does not limit the present disclosure in any way.

In general, the device 600 shown in FIG. 6 includes a housing 602 providing a cavity configured to hold a cartridge having a volatile material therein (not shown). The housing 602 is also configured to hold therein an electrical assembly (not shown), and optionally other elements and components. In some implementations, the electrical assembly is configured to control a release of the volatile material from the cartridge. The electrical assembly is configured to interact with a power outlet of a vehicle via socket contacts 604.

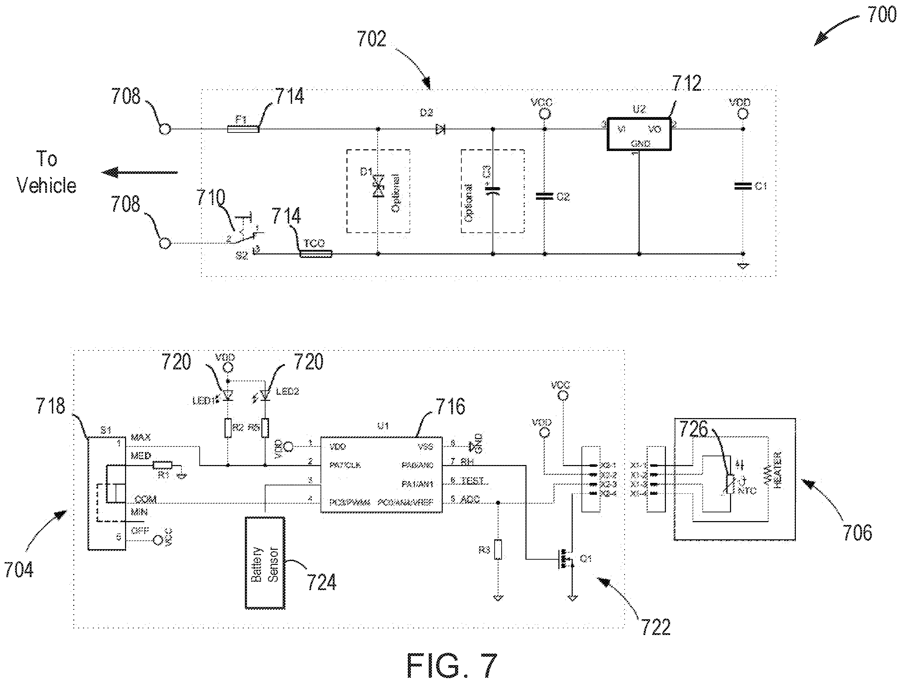

Referring specifically to FIG. 7, an example electrical assembly 700 for use in the device 600 is shown. The electrical assembly 700 includes a power stage 702, a controller stage 704, and a heating element 706. In particular, the power stage 702 is configured to receive power from a vehicle's battery by way of input leads 708 that are connected to the socket contacts 604 shown in FIG. 6. The power stage 702 is also configured to manage the received power to operate various electrical components of the electrical assembly 700, such as the heating element 706.

As shown in FIG. 7, the power stage 702 may include a pushbutton switch 710 having an "on" and an "off" position, for example, and a voltage regulator 712. The power stage 702 may also include a number of other electrical components, including capacitors, resistors, inductors, diodes, and so forth. In addition, as shown in FIG. 7, the power stage 702 may also include a number of fuses 714, such as electrical and thermal fuses, for protecting circuit components of the electrical assembly 700 in case of electrical or thermal spikes, transients, or overload.

Still referring to FIG. 7, the control stage 704 includes a processor 716 (e.g. a microcontroller) programmed to control the operation of the heating element 706, and other electrical components. In addition, the control stage 704 also includes a slide switch 718 for selecting the mode of operation. Specifically, the power switch 718 activates inputs to the processor 716 to indicate a target temperature for the heating element 706. By way of example, the slide switch 718 may include an "off" position, and a number of "on" positions, such as a "low," "medium," and "high" position, indicating an intensity level for dispersing volatile material. The position of the slide switch 718 may be indicated by LEDs 720 included in the control stage 704 circuitry, as shown in FIG. 7. In some implementations, the same or different LEDs 720 may additionally, or alternatively, indicate an "OFF" or "ON" state of the vehicle.

When the pushbutton switch 710 and slide switch 718 are activated to an "on" position, the processor 716 can direct electric current to flow to the heating element 706, using activation element 722 and power supplied by the power stage 702. In some aspects, a PWM algorithm may be used by the processor 716 to allow the heating element 706 to heat up quickly, which in turn would allow a faster fragrance or volatile material release. In some implementations, the processor 716 may also be programmed such that if the slide switch 718 is inadvertently moved to an intermediate position that is a position between allowable settings, as described above, the electrical assembly 700, or portions thereof, may be disabled, to avoid unpredictable behavior.

As shown in FIG. 7, the control stage 704 may include a battery sensor 724 in communication with the processor 716. The battery sensor 724 is configured to detect battery voltage of the vehicle's battery, as described. The processor 716 is configured to control a sampling of the battery voltage detected by the battery sensor 724, and determine an operational state of the vehicle using the battery voltage, as described. Based on the operational state, the processor 716 can affect the operation of the heating element 706, as well as other electrical components. In particular, the processor 716 may generate and send a control signal to the activation element 722, which would either prevent or allow power being provided to the heating element 706. For example, the processor 716 may deenergize the heating element 706 when a vehicle is in an "OFF" state. A determination of an "ON" or "OFF" state may be reported to a user, for example, using the LEDs 720.

In some modes of operation, the processor 716 may temporarily energize the heating element 706 to apply a load to the vehicle's battery. This may be desirable in the case that the battery voltage detected using the battery sensor 724 is below a predetermined threshold (e.g. about 13.0V). As described with reference to FIG. 4, applying a load (in this case the heating element 706) and monitoring battery voltage for a predetermined period of time allows for determining the operational state of the vehicle. As described, the period of time may depend on the nature of the load (e.g. power draw) and other factors.

In some embodiments, the heating element 706 may include a thermistor 726 that is coupled to the processor 716, and allows the processor 716 to shut off specific electronic components, including the heating element 706, for a predetermined amount of time if a predetermined temperature is exceeded.

Although a particular implementation is shown in FIG. 7 for the power stage 702 and controller stage 704 for managing and controlling power provided to the heating element 706, any number of modifications and variations are possible to provide functionalities as described above, as well as other functionalities. Additionally, the heating element 706 is shown to include a single resistive wire, yet it may be readily appreciated that any variation, such as two or more resistive wires, in accordance with the present disclosure may also be possible.

### INDUSTRIAL APPLICABILITY

Devices and methods are presented that provide a novel approach for controlling electronic devices for use in a vehicle based on the operational state of the vehicle.

Numerous modifications to the present invention will be apparent to those skilled in the art in view of the foregoing description. Accordingly, this description is to be construed as illustrative only and is presented for the purpose of enabling those skilled in the art to make and use the invention and to teach the best mode of carrying out same.

## Claims

1. A method for controlling an electronic device (300) for use in a vehicle (350), the method comprising:
detecting a battery voltage of a vehicle's battery (402) using a battery sensor (304) connected thereto;
determining an operational state of the vehicle (350) using the battery voltage, wherein the battery voltage is compared to a predetermined threshold;
applying a load to the vehicle's battery (402) if the battery voltage is below the predetermined threshold value;
identifying a change in the battery voltage due to the load;
determining the operational state of the vehicle (350) based on the change in the battery voltage; and
controlling an electronic device (300) coupled to the vehicle's battery (402) in accordance with the operational state of the vehicle (350).

2. The method of claim 1, wherein the method further comprises sampling the battery voltage detected by the battery sensor (304) intermittently or continuously.

3. The method of claim 1, wherein the method further comprises monitoring the battery voltage for a predetermined period of time if the battery voltage is below the predetermined threshold.

4. The method of claim 1, wherein applying the load to the vehicle's battery (402) comprises electrically connecting a heating element (706) of the electronic device (300) to the vehicle's battery (402).

5. The method of claim 1, wherein the method further comprises controlling at least one device function of the electronic device (300).

6. An electronic device (300) for use in a vehicle (350), the device comprising:
a battery sensor (304) configured to detect a battery voltage when connected to a vehicle's battery (402);
a load circuit (410) configured to apply a load to the vehicle's battery (402); and
a processor (306) in communication with the battery sensor (304) that is configured to:
sample the battery voltage detected by the battery sensor (304),
determine an operational state of the vehicle (350) using the battery voltage,
control the load circuit (410) to apply the load, and identify a change in the battery voltage due to the load using the battery voltage detected by the battery sensor (304),
determine the operational state of the vehicle (350) based on the change in the battery voltage, and
control the electronic device (300) in accordance with the operational state of the vehicle (350).

7. The device of claim 6, wherein the processor (306) is further configured to sample the battery voltage intermittently or continuously.

8. The device of claim 6, wherein the processor (306) is further configured to compare the battery voltage to a predetermined threshold to determine the operational state of the vehicle (350).

9. The device of claim 8, wherein the processor (306) is further configured to sample the battery voltage for a predetermined period of time if the battery voltage is below the predetermined threshold.

10. The device of claim 6, wherein the load circuit (410) comprises a heating element (706).

11. The device of claim 6, wherein the processor (306) is further configured to control at least one function module (308) in the electronic device (300), wherein each of the at least one function module (308) is configured to carry out a function in the electronic device (300).

12. The device of claim 6, where the device is a volatile emitting device (600) comprising:
a cartridge having a volatile material therein;
an electrical assembly (700) comprising the battery sensor (304; 724), the processor (306; 716) and the load circuit (410); and
a housing (602) having a cavity configured to hold the cartridge and electrical assembly (700).

## Patentansprüche

1. Ein Verfahren zum Steuern einer elektronischen Vorrichtung (300) zur Verwendung in einem Fahrzeug (350), wobei das Verfahren Folgendes beinhaltet:
Erfassen einer Batteriespannung einer Fahrzeugbatterie (402) unter Verwendung eines damit verbundenen Batteriesensors (304);
Bestimmen eines Betriebszustands des Fahrzeugs (350) unter Verwendung der Batteriespannung, wobei die Batteriespannung mit einem vorbestimmten Schwellenwert verglichen wird;
Anlegen einer Last an die Fahrzeugbatterie (402), wenn die Batteriespannung unter dem vorbestimmten Schwellenwert liegt;
Identifizieren einer Änderung der Batteriespannung aufgrund der Last;
Bestimmen des Betriebszustands des Fahrzeugs (350) basierend auf der Änderung der Batteriespannung; und
Steuern einer elektronischen Vorrichtung (300), die mit der Fahrzeugbatterie (402) gekoppelt ist, gemäß dem Betriebszustand des Fahrzeugs (350).

2. Verfahren gemäß Anspruch 1, wobei das Verfahren ferner das intermittierende oder kontinuierliche Abtasten der von dem Batteriesensor (304) erfassten Batteriespannung beinhaltet.

3. Verfahren gemäß Anspruch 1, wobei das Verfahren ferner das Überwachen der Batteriespannung für einen vorbestimmten Zeitraum beinhaltet, wenn die Batteriespannung unter dem vorbestimmten Schwellenwert liegt.

4. Verfahren gemäß Anspruch 1, wobei das Anlegen der Last an die Fahrzeugbatterie (402) das elektrische Verbinden eines Heizelements (706) der elektronischen Vorrichtung (300) mit der Fahrzeugbatterie (402) beinhaltet.

5. Verfahren gemäß Anspruch 1, wobei das Verfahren ferner das Steuern mindestens einer Vorrichtungsfunktion der elektronischen Vorrichtung (300) beinhaltet.

6. Eine elektronische Vorrichtung (300) zur Verwendung in einem Fahrzeug (350), wobei die Vorrichtung Folgendes beinhaltet:
einen Batteriesensor (304), der konfiguriert ist, um eine Batteriespannung zu erfassen, wenn er mit einer Fahrzeugbatterie (402) verbunden ist;
eine Lastschaltung (410), die konfiguriert ist, um eine Last an die Fahrzeugbatterie (402) anzulegen; und
einen Prozessor (306) in Kommunikation mit dem Batteriesensor (304), der für Folgendes konfiguriert ist:
Abtasten der von dem Batteriesensor (304) erfassten Batteriespannung,
Bestimmen eines Betriebszustands des Fahrzeugs (350) unter Verwendung der Batteriespannung,
Steuern der Lastschaltung (410), um die Last anzulegen, und Identifizieren einer Änderung der Batteriespannung aufgrund der Last unter Verwendung der von dem Batteriesensor (304) erfassten Batteriespannung,
Bestimmen des Betriebszustands des Fahrzeugs (350) basierend auf der Änderung der Batteriespannung, und
Steuern der elektronischen Vorrichtung (300) gemäß dem Betriebszustand des Fahrzeugs (350).

7. Vorrichtung gemäß Anspruch 6, wobei der Prozessor (306) ferner konfiguriert ist, um die Batteriespannung intermittierend oder kontinuierlich abzutasten.

8. Vorrichtung gemäß Anspruch 6, wobei der Prozessor (306) ferner konfiguriert ist, um die Batteriespannung mit einem vorbestimmten Schwellenwert zu vergleichen, um den Betriebszustand des Fahrzeugs (350) zu bestimmen.

9. Vorrichtung gemäß Anspruch 8, wobei der Prozessor (306) ferner konfiguriert ist, um die Batteriespannung für einen vorbestimmten Zeitraum abzutasten, wenn die Batteriespannung unter dem vorbestimmten Schwellenwert liegt.

10. Vorrichtung gemäß Anspruch 6, wobei die Lastschaltung (410) ein Heizelement (706) beinhaltet.

11. Vorrichtung gemäß Anspruch 6, wobei der Prozessor (306) ferner konfiguriert ist, um mindestens ein Funktionsmodul (308) in der elektronischen Vorrichtung (300) zu steuern, wobei jedes des mindestens einen Funktionsmoduls (308) konfiguriert ist, um eine Funktion in der elektronischen Vorrichtung (300) auszuführen.

12. Vorrichtung gemäß Anspruch 6, wobei die Vorrichtung eine flüchtige emittierende Vorrichtung (600) ist, die Folgendes beinhaltet:
eine Kartusche mit einem flüchtigen Material darin;
eine elektrische Baugruppe (700), die den Batteriesensor (304; 724), den Prozessor (306; 716) und die Lastschaltung (410) beinhaltet; und
ein Gehäuse (602) mit einem Hohlraum, der konfiguriert ist, um die Kartusche und die elektrische Baugruppe (700) zu halten.

## Revendications

1. Un procédé pour la commande d'un dispositif électronique (300) destiné à être utilisé dans un véhicule (350), le procédé comprenant :
la détection d'une tension de batterie d'une batterie (402) du véhicule à l'aide d'un capteur de batterie (304) connecté à celle-ci ;
la détermination d'un état de fonctionnement du véhicule (350) à l'aide de la tension de batterie, où la tension de batterie est comparée à un seuil prédéterminé ;
l'application d'une charge à la batterie (402) du véhicule si la tension de batterie est en dessous de la valeur de seuil prédéterminé ;
l'identification d'une variation de la tension de batterie due à la charge ;
la détermination de l'état de fonctionnement du véhicule (350) sur la base de la variation de la tension de batterie ; et
la commande d'un dispositif électronique (300) couplé à la batterie (402) du véhicule conformément à l'état de fonctionnement du véhicule (350).

2. Le procédé de la revendication 1, le procédé comprenant en outre l'échantillonnage de la tension de batterie détectée par le capteur de batterie (304) de façon intermittente ou de façon continue.

3. Le procédé de la revendication 1, le procédé comprenant en outre la surveillance de la tension de batterie pendant une durée prédéterminée si la tension de batterie est en dessous du seuil prédéterminé.

4. Le procédé de la revendication 1, où l'application de la charge à la batterie (402) du véhicule comprend le fait de connecter électriquement un élément chauffant (706) du dispositif électronique (300) à la batterie (402) du véhicule.

5. Le procédé de la revendication 1, le procédé comprenant en outre la commande d'au moins une fonction de dispositif du dispositif électronique (300).

6. Un dispositif électronique (300) destiné à être utilisé dans un véhicule (350), le dispositif comprenant :
un capteur de batterie (304) configuré pour détecter une tension de batterie lorsqu'il est connecté à la batterie (402) d'un véhicule ;
un circuit de charge (410) configuré pour appliquer une charge à la batterie (402) du véhicule ; et
un processeur (306) en communication avec le capteur de batterie (304) qui est configuré pour :
échantillonner la tension de batterie détectée par le capteur de batterie (304),
déterminer un état de fonctionnement du véhicule (350) à l'aide de la tension de batterie,
commander le circuit de charge (410) pour qu'il applique la charge, et identifier une variation de la tension de batterie due à la charge à l'aide de la tension de batterie détectée par le capteur de batterie (304),
déterminer l'état de fonctionnement du véhicule (350) sur la base de la variation de la tension de batterie, et
commander le dispositif électronique (300) conformément à l'état de fonctionnement du véhicule (350).

7. Le dispositif de la revendication 6, où le processeur (306) est configuré en outre pour échantillonner la tension de batterie de façon intermittente ou de façon continue.

8. Le dispositif de la revendication 6, où le processeur (306) est configuré en outre pour comparer la tension de batterie à un seuil prédéterminé afin de déterminer l'état de fonctionnement du véhicule (350).

9. Le dispositif de la revendication 8, où le processeur (306) est configuré en outre pour échantillonner la tension de batterie pendant une durée prédéterminée si la tension de batterie est en dessous du seuil prédéterminé.

10. Le dispositif de la revendication 6, où le circuit de charge (410) comprend un élément chauffant (706).

11. Le dispositif de la revendication 6, où le processeur (306) est configuré en outre pour commander au moins un module de fonction (308) dans le dispositif électronique (300), où chaque module de l'au moins un module de fonction (308) est configuré pour exécuter une fonction dans le dispositif électronique (300).

12. Le dispositif de la revendication 6, le dispositif étant un dispositif émetteur de substance volatile (600) comprenant :
une cartouche contenant une substance volatile ;
un ensemble électrique (700) comprenant le capteur de batterie (304 ; 724), le processeur (306 ; 716) et le circuit de charge (410) ; et
un boîtier (602) possédant une cavité configurée pour renfermer la cartouche et l'ensemble électrique (700).
